# EUROPEAN PATENT APPLICATION

(11) **EP 4 722 718 A1**
(43) Date of publication of application: **08.04.2026**
(21) Application number: 24900779.0
(22) Date of filing: 22.08.2024
(51) Int. Cl.: G01N 33/44

(54) **METHOD FOR PREDICTING NECK-IN PROPERTY OF POLYLACTIDE RESIN**

(30) Priority: 06.12.2023 KR 20230175672
(71) Applicant: LG Chem, Ltd., Seoul 07336 (KR)
(72) Inventor: CHO, Jeong Hun, Daejeon 34122 (KR); PARK, Junki, Daejeon 34122 (KR); LEE, Daewoong, Daejeon 34122 (KR); KIM, Sehoon, Daejeon 34122 (KR); LEE, Myunghan, Daejeon 34122 (KR); OH, Wan Kyu, Daejeon 34122 (KR)
(74) Representative: Goddar, Heinz J.
(86) International application number: PCT/KR2024/012485
(87) International publication number: WO 2025/121591

(57) **Abstract**

The present disclosure provides a method for predicting or evaluating neck-in property of a film produced from a polylactide resin, which is simple and high in prediction accuracy and thus can replace the actual measurement method, thereby determining film properties for new structures in advance.

## Description

### [TECHNICAL FIELD]

### CROSS-REFERENCE TO RELATED APPLICATION(S)

This application claims priority from and the benefit of Korean Patent Application No. 10-2023-0175672 filed on December 6, 2023 in the Korean Intellectual Property Office, the disclosure of which is incorporated herein by reference in its entirety.

The present disclosure relates to a method for predicting neck-in property of polylactide resin.

### [BACKGROUND]

Polylactic acid (PLA) is a plant-derived resin obtained from plants such as corn, and is attracting attention as an environment-friendly material having biodegradable properties. Unlike petroleum-based resins such as polystyrene resins, polyvinyl chloride resins, polyethylene resins, etc., which are currently used, polylactic acid has effects of preventing depletion of petroleum-based resources and suppressing carbon dioxide emissions, and so it can reduce environmental pollution, which is a drawback of petroleum-based plastic products. As environmental pollution caused by waste plastics and the like has emerged as social problems, efforts have been made to expand the scope of application of polylactic acid to the fields of the products where general plastics (petroleum-based resins) were used, such as food packaging materials and containers, and electronic product cases.

However, since polylactide resin has a low melt strength and melt elasticity due to its structural characteristics, the molding process stability is reduced during film production. In particular, when the production speed is increased in order to improve productivity, the neck-in phenomenon increases, and there is a limit to thinning the film thickness in consideration of the processability. In addition, there is a disadvantage that the edge weave becomes intense in the molding process, and the width and thickness deviation of the produced film become intense.

As a proposal to improve the above-mentioned disadvantages, many attempts have been made to develop new polylactide resins having a branched structure and improve polymer chain entanglements. One of them is to add an epoxy-based branching agent to a polylactide resin to improve melt strength. Since the carboxyl and epoxy groups in the end groups of polylactide resin mainly react to form a branched structure, the polylactide resin may show significant differences in reactivity depending on its acid value (influence of end groups due to additives, initiators, etc.) and purity (D content). Therefore, the tendency to branch is diverse, and it is not easy to grasp the extent to which physical properties ensure processing stability while improving the neck-in property until the actual product is evaluated.

Therefore, the present disclosure provides a method for predicting or evaluating neck-in property of a film produced from a polylactide resin, which is simple and high in prediction accuracy and thus can replace the actual measurement method, thereby determining film properties for new structures in advance.

### [DETAILED DESCRIPTION OF THE INVENTION]

### [Technical Problem]

It is an object of the present disclosure to provide a method for predicting neck-in property of polylactide resin.

### [Technical Solution]

To achieve the above objects, provided therein is a method for predicting neck-in property of a polylactide resin, the method comprising the steps of:
1) measuring the following physical properties A and B for a plurality of polylactide resins respectively (step 1);
   - Physical property A: Tan δ (@0.4 rad/s)
   - Physical property B: η" (@0.3 rad/s)
2) measuring a Neck-in value (N) for the plurality of polylactide resins (step 2); and
3) performing a regression analysis on the Neck-in value (N) according to the measured values of physical properties A and B to derive a Neck-in prediction equation of the polylactide resin (step 3).

The term "polylactide resin" as used here refers to comprehensively include a single polymer or a copolymer containing the following repeating units.

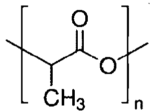

The polylactide resin can be produced by a method including a step of forming the repeating unit by ring-opening polymerization of a lactide monomer, and the polymer after the ring-opening polymerization and the process of forming the repeating units are completed can be referred to as the "polylactide resin".

In this case, the term "lactide monomer" can be defined as follows. Typically, lactides can be classified into L-lactide consisting of L-lactic acid, D-lactide consisting of D-lactic acid, and meso-lactide consisting of one L-type and one D-type. Also, a mixture of L-lactide and D-lactide in a weight ratio of 50:50 is referred to as D,L-lactide or rac-lactide. Among these lactides, the polymerization proceeding only with either of L-lactide and D-lactide that have a high level of optical purity is known to yield an L- or D-polylactide (PLLA or PDLA) with a high level of stereoregularity. Such polylactides have a faster crystallization rate and a higher crystallization degree than a polylactide having a low level of optical purity. However, the term "lactide monomer" as used herein is defined to include all types of lactides regardless of the difference in the characteristics of lactides depending on their types and the difference in the characteristics of the polylactide resin obtained therefrom.

Meanwhile, the plurality of polylactide resins each have a weight average molecular weight of 70,000 to 400,000. Preferably, the polylactide resin according to the present disclosure has a weight average molecular weight of 80,000 or more, 90,000 or more, or 100,000 or more; and 300,000 or less, 250,000 or less, or 200,000 or less.

Also, the plurality of polylactide resins have a number average molecular weight of 50,000 to 100,000. Preferably, the polylactide resin according to the present disclosure has a number average molecular weight of 55,000 or more, or 60,000 or more; 90,000 or less, 85,000 or less, or 80,000 or less.

In addition, each of the plurality of polylactide resins may further include an additive. The additive may include a polylactide oligomer, a branching agent, and the like. For example, some polylactide resins may not include any other components in addition to the polylactide resin, and some may include an additive. In addition, it is possible to predict how the additive used affects Neck-in properties through a method in which the polylactide resin itself uses the same resin and only the type or content of additives is changed.

The present disclosure relates to a method for predicting or evaluating the Neck-in properties of a film prepared from the polylactide resin, which is simple and high in prediction accuracy. For this purpose, the present disclosure measures the rheological properties of the physical properties A and B for the polylactide resin.

The physical property A is Tan delta, and the tan delta value for each frequency section can be confirmed through the measurement of the rheological properties. A specific measurement method therefor is embodied in Examples described hereinafter. In the low frequency (0.3 ~ 0.4 rad/s) range, there is a correlation in which the Neck-in decreases as Tan delta decreases when compared to the actual Neck-in value within the processing conditions.

The physical property B is η", and the results of η' and η" for each frequency section can be confirmed through the measurement of rheological properties, and can be obtained by plotting the Cole-Cole Plot, and a specific measurement method therefor is embodied in Examples described below. In the low frequency (0.3-0.4 rad/s) range, there was a correlation in which Neck-in decreased as η" increased compared to the actual Neck-in value within the processing conditions.

Therefore, the physical properties A and B may be measured for a plurality of polylactide resins (step 1), the Neck-in value (N) may be measured for the plurality of polylactide resins (step 2), and the regression analysis on Neck-in value (N) according to the measured values of physical properties A and B may be performed to derive a Neck-in prediction equation of the polylactide resin.

The plurality of polylactide resins means at least two types of polylactide resins, and it is preferable that the types are many in consideration of the correlation coefficient of the prediction equation. Preferably, the plurality of polylactide resins are preferably 5 or more types, 6 or more types, 7 or more types, 8 or more types, 9 or more types, or 10 or more types, and 50 or less, 40 or less types, 30 or less types, or 20 or less types of polylactide resins.

Preferably, the Neck-in prediction equation of the polylactide resin is as shown in the following Mathematical Equation 1: $N = \left(0.077*A\right) - \left(0.085*B\right) + 27.69$
in Mathematical Equation 1,
A means Tan δ (@0.4 rad/s), and B means η" (@0.3 rad/s).

As shown in Examples described below, the prediction equation obtained according to the present disclosure has a high correlation with the actual measurement value, and the difference between the value calculated by the obtained prediction equation and the actual measurement value is very small. Therefore, according to the present disclosure, it is possible to predict film properties simply and with high prediction accuracy even without producing an actual film.

### [Advantageous Effects]

As descried above, the present disclosure provides a method for predicting or evaluating neck-in property of a film produced from a polylactide resin, which is simple and high in prediction accuracy and thus can replace the actual measurement method, thereby determining film properties for new structures in advance.

### [BRIEF DESCRIPTION OF THE DRAWIINGS]

FIG. 1 shows the results of mapping the two factors measured in Experimental Example 1.
FIG. 2 shows the method of obtaining the Neck-in prediction equation in Experimental Example 1.

### [DETAILED DESCRIPTION OF THE EMBODIMENTS]

Hereinafter, exemplary embodiments of the present disclosure will be described in more detail in the following Examples. However, the following Examples are for illustrative purposes only, and the content of the present invention is not limited by them.

### Preparation Example 1: Preparation of PT-SA

A PLA oligomer was prepared using pentaerythritol (PT) as an initiator. Specifically, lactide: PT = 100:1 (molar ratio) was added to a 500 mL reactor to make a total of 75 g, and Sn(Oct)₂ catalyst (0.075 g) was added thereto, and then reacted at 180°C for 5 hours to prepare an oligomer. In order to replace the hydroxyl end group of the prepared oligomer with a carboxyl group (COOH), the mole number was calculated from the amount of lactide added (g) and the number average molecular weight (g/mol) of the prepared oligomer, and the total hydroxyl end group of the prepared oligomer was calculated taking into account the number of end groups (4) of the initiator (PT). Then, 2 equivalents of succinic anhydride (SA) relative to the total hydroxyl end groups of the above oligomer were added in one pot, and the reaction was allowed to proceed for an additional 3 hours. After the reaction was completed, the PLA oligomer prepared in the CHCl₃/MeOH solvent system was precipitated/separated to remove unreacted SA and remaining impurities. The final product was obtained by vacuum drying at 45°C for 12 hours, and was named 'PT-SA'.

The weight average molecular weight of 'PT-SA', which is the prepared PLA oligomer 'PT-SA', was measured. Specifically, the weight average molecular weight (Mw) was measured using GPC (Gel Permeation Chromatography) device, and the specific measurement conditions were as follows, and the weight average molecular weight measured thereby was approximately 40,000.
- Column: PLgel Mixed E x 2
- Solvent: THF
- Flow rate: 0.7 mL/min
- Sample concentration: 3.0 mg/mL
- Injection volume: 100 µl
- Column temperature: 40°C
- Detector: Waters 2414 RID
- Standard: PS (polystyrene)

### Example: Preparation of Polylactide resin

Reactive extrusion was carried out for the components listed in Table 1 below using 19Ψ extruder (BA-19, Bautek).

Specifically, 1 kg of polylactide resin (Anhui BBCA Biochemical & Futerro Corp., PLA, FY201; FY202; FY212; FY601; FY801) was mixed with the components (epoxy type branching agent (Joncryl ADR 4468), or PT-SA) listed in Table 1 below, and extruded at a screw speed of 200 rpm, and the maximum temperature value was adjusted (190 to 230°C) to obtain PLA pellets. The number average molecular weight and weight average molecular weight of the obtained PLA pellets were measured as follows, and shown in Table 1 below.
- Column: PL mixed B x 2
- Solvent: THF
- Flow rate: 1.0 ml/min
- Sample concentration: 1.5 mg/ml
- Injection volume: 100 µl
- Column temperature: 40°C
- Detector: Waters 2414 RID
- Standard: PS (polystyrene)

**[Table 1]**

| Sample | #1 | #2 | #3 | #4 | #5 | #6 | #7 | #8 |
|---|---|---|---|---|---|---|---|---|
| PLA | FY201 | FY201 | FY201 | FY202 | FY202 | FY202 | FY212 | FY212 |
| Additive | Joncryl 1.0 | Joncryl 1.0 | Joncryl 1.0 | Joncryl 1.0 | Joncryl 1.0 | Joncryl 1.0 | Joncryl 1.0 | Joncryl 1.0 |
| | | | | | | PT-SA 5.0 | | |
| F/R | 5 | 5 | 5 | 5 | 5 | 5 | 5 | 5 |
| RPM | 200 | 200 | 200 | 200 | 200 | 200 | 200 | 200 |
| Zone 7 (°C) | 130 | 130 | 130 | 130 | 130 | 130 | 110 | 130 |
| Zone 6 (°C) | 170 | 190 | 200 | 190 | 200 | 200 | 170 | 200 |
| Zone 5 (°C) | 190 | 210 | 230 | 210 | 230 | 230 | 190 | 230 |
| Zone 4 (°C) | 190 | 210 | 230 | 210 | 230 | 230 | 190 | 230 |
| Zone 3 (°C) | 190 | 210 | 230 | 210 | 230 | 230 | 190 | 230 |
| Zone 2 (°C) | 190 | 210 | 230 | 210 | 230 | 230 | 190 | 230 |
| Zone 1 (°C) | 190 | 210 | 230 | 210 | 230 | 230 | 190 | 230 |
| Header (°C) | 190 | 210 | 230 | 210 | 210 | 210 | 210 | 210 |
| Mn | 66,184 | 61,373 | 63,963 | 63,950 | 63,017 | 60,251 | 76,588 | 74,391 |
| Mw | 121,979 | 117,746 | 128,905 | 128,895 | 132,086 | 139,512 | 162,102 | 190,793 |
| PDI | 2 | 1.92 | 2.02 | 2.02 | 2.1 | 2.32 | 2.12 | 2.57 |

In addition, the following four types of polylactide resins were used in Experiments below.
#9: NW 4032D (NatureWorks)
#10: FY601 (Anhui BBCA Biochemical & Futerro PLA Corp.)
#11: FY604 (Anhui BBCA Biochemical & Futerro PLA Corp)
#12: FY801 (Anhui BBCA Biochemical & Futerro PLA Corp)

### Experimental Example 1

### 1) Neck-in Measurement of Polylactide Resin

The obtained pellets were vacuum-dried at 85°C for 4 hours or more, and PLA films were produced through T-die extrusion molding (Eurotech benchtop monolayer cast film device, Screw Ψ17.5mm, T-die width 120 mm).

Neck-in value was measured by the film width (X) produced under the same conditions of calendar speed of 3.0 M/m and haul-off speed of 4.5 M/m, and the results are shown in Table 2 below.

Neck-in evaluation: $\left(\left(T-die width\right)-\left(Film width\right)\right) / 2 = \left(120-X\right) / 2$

**[Table 2]**

| Sampl e | T-die condition | | | | | Calendar | Haul-off | Film evaluation | | |
|---|---|---|---|---|---|---|---|---|---|---|
| | Scre w | Cyl 1 | Cyl 1 | Head | Lip | M/m | M/m | Film thickne ss | Film width | Neck-in evaluati on |
| | (rpm) | (°C) | (°C) | (°C) | (°C) | | | (µm) | (mm) | (mm) |
| #1 | 50 | 150 | 200 | 200 | 190 | 3 | 4.5 | 35-69 | 24-65 | 48 |
| #2 | 50 | 150 | 200 | 200 | 190 | 3 | 4.5 | 37-68 | 24-48 | 48 |
| #3 | 50 | 150 | 200 | 200 | 190 | 3 | 4.5 | 35-47 | 45-62 | 37.5 |
| #4 | 50 | 150 | 200 | 200 | 190 | 3 | 4.5 | 47-68 | 48-58 | 36 |
| #5 | 50 | 150 | 200 | 200 | 190 | 3 | 4.5 | 22-73 | 35-50 | 42.5 |
| #6 | 50 | 150 | 200 | 200 | 190 | 3 | 4.5 | 38-47 | 64-73 | 28 |
| #7 | 50 | 150 | 200 | 200 | 190 | 3 | 4.5 | 22-34 | 94-95 | 13 |
| #8 | 50 | 110 | 200 | 200 | 190 | 3 | 4.5 | 25-31 | 94-95 | 13 |
| #9 | 50 | 110 | 200 | 200 | 190 | 3 | 4.5 | 18-23 | 56-59 | 32 |
| #10 | 50 | 150 | 200 | 200 | 190 | 3 | 4.5 | 17-36 | 44-54 | 38 |
| #11 | 50 | 110 | 200 | 200 | 190 | 3 | 4.5 | 22-26 | 50-58 | 35 |
| #12 | 50 | 110 | 200 | 200 | 190 | 3 | 4.5 | 15-23 | 50-70 | 35 |

### 2) Derivation of Neck-in Prediction Equation for Polylactide Resin

Using ARES-G2 Rheometer (Strain-Controlled Type) from TA Instruments, the rheological properties were evaluated on the basis of the geometry of 25 mm diameter parallel plate, 1 mm gap. The PLA sample was filled and added into the geometry size and measured, and the appropriate strain according to the sample was set through Amplitude Sweep at the initial measurement temperature of 210°C. Based on the set strain (5-10%), 0.1 rad/s~500 rad/s Angular Frequency Sweep was performed at the same measurement temperature of 210°C. From this, the following two factors were derived.

### (i) Tan delta

The tan delta value for each frequency section was confirmed through rheological measurement. When compared with the Neck-in test results within the processing conditions in the low frequency (0.3-0.4 rad/s) range reflecting the structural (LCB) effect, there was a correlation in which Neck-in decreased as Tan delta decreased.

### (ii) η"

The η', η" results for each frequency section were confirmed through the same measurement method, and Cole-Cole Plot could be plotted. η" in the Cole-Cole Plot showed a tendency to increase according to the PLA structural change (LCB). When compared with the Neck-in test results within the processing conditions in the low frequency (0.3-0.4 rad/s) range reflecting the structural (LCB) effect, there was a correlation in which Neck-in decreased as η" increased.

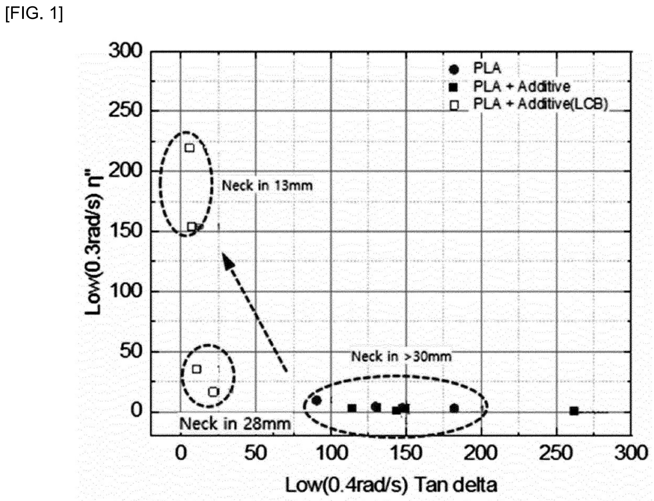

The above two types of significant factors had a high correlation with Neck-in, and mapping between significant factors could be performed, which was illustrated in FIG. 1.

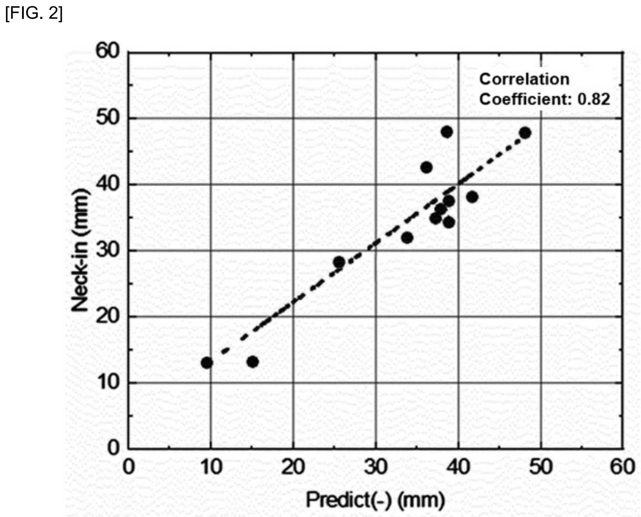

The above two type of significant factors have a high correlation with Neck-in, and based on this, Neck-in prediction using the rheological factor is possible. The data is summarized in Table 3 below, and the prediction equation between factors can be constructed through a linear regression model, as illustrated in FIG. 2, and the correlation coefficient between the prediction equation and Neck-in was 0.82.

**[Table 3]**

| Sample | Low (0.4rad/s) Tan delta | Low (0.3rad/s) η* | Neck-in actual measurement value | Neck-in prediction value |
|---|---|---|---|---|
| | @210°C | @210°C | @3M/m | @3M/m |
| #1 | 261.884 | 0.10696 | 48 | 47.8 |
| #2 | 143.873 | 0.10696 | 48 | 38.8 |
| #3 | 149.331 | 2.09484 | 37.5 | 39 |
| #4 | 131.003 | 2.87326 | 36 | 37.5 |
| #5 | 114.118 | 2.84014 | 42.5 | 36.2 |
| #6 | 10.4315 | 35.0199 | 28 | 25.5 |
| #7 | 5.83055 | 219.489 | 13 | 9.5 |
| #8 | 6.98884 | 154.057 | 13 | 15.1 |
| #9 | 90.3746 | 9.45854 | 32 | 33.8 |
| #10 | 182.254 | 2.6088 | 38 | 41.5 |
| #11 | 147.532 | 2.9928 | 35 | 38.8 |
| #12 | 129.684 | 4.26071 | 35 | 37.3 |

$Neck - in \left(prediction equation\right) = 0.077 * A - 0.085 * B + 27.69$
in the above equation,
A is Tan delta (@0.4 rad/s), and
B is η" (@0.3 rad/s).

### Experimental Example 2

In order to evaluate the prediction equation obtained in Experimental Example 1, a polylactide resin composition was prepared in the same manner as in the preparation method of the previous sample #7, but by using 0.5 phr of Joncryl ADR 4468. The weight average molecular weight and number average molecular weight thereof were 162,102 and 76,588, respectively.

For the polylactide resin composition above, Tan delta and η" were measured in the same manner as in Example 1, and were measured as 21.5255 and 16.431, respectively. These values were substituted into the prediction equation obtained in Experimental Example 1 to predict the Neck-in value as follows. $\begin{matrix}Neck - in = 0.077 * \left(21.5255\right) - 0.085 * \left(16.431\right) + 27.69 \\ = 27.95\end{matrix}$

For the above polylactide resin composition, the Neck-in actual measurement value was measured in the same manner as in Example 1, and was found to be 27.5, which was confirmed to be almost the same as the value obtained in the above prediction equation.

## Claims

1. A method for predicting neck-in property of a polylactide resin, the method comprising the steps of:
1) measuring the following physical properties A and B for a plurality of polylactide resins respectively (step 1);
- Physical property A: Tan δ (@0.4 rad/s)
- Physical property B: η" (@0.3 rad/s)
2) measuring a Neck-in value (N) for the plurality of polylactide resins (step 2); and
3) performing a regression analysis on the Neck-in value (N) according to the measured values of physical properties A and B to derive a Neck-in prediction equation of the polylactide resin (step 3).

2. The prediction method of claim 1, wherein:
the Neck-in prediction equation of the polylactide resin is the following Mathematical Equation 1: $N = \left(0.077*A\right) - \left(0.085*B\right) + 27.69$
in Mathematical Equation 1,
A means Tan δ (@0.4 rad/s), and
B means η" (@0.3 rad/s).

3. The prediction method of claim 1, wherein:
the plurality of polylactide resins each have a weight average molecular weight of 70,000 to 400,000,

4. The prediction method of claim 1, wherein:
the plurality of polylactide resins have a number average molecular weight of 50,000 to 100,000.

5. The prediction method of claim 1, wherein:
some of the plurality of polylactide resins further comprise an additive.

6. The prediction method of claim 1, wherein:
the plurality of polylactide resins are 10 types to 20 types of resins.
